# EUROPEAN PATENT SPECIFICATION

(11) **EP 0 280 814 B1**
(45) Date of publication and mention of the grant of the patent: **31.03.1993**
(21) Application number: 87310913.6
(22) Date of filing: 11.12.1987
(51) Int. Cl.: G01F 1/74, G01F 1/704, G01F 1/64

(54) **Apparatus and method for measuring the flow characteristics of a petroleum stream**
Gerät und Verfahren zum Messen der Strömungscharakteristika einer Petroleumströmung
Appareil et procédé pour la mesure d'une caractéristique fluidique d'un courant de pétrole

(30) Priority: 05.01.1987 US 337
(43) Date of publication of application: 07.09.1988
(73) Proprietor: TEXACO DEVELOPMENT CORPORATION, White Plains, New York 10650 (US)
(72) Inventor: Ekrann, Steinar, N-4000 Stavanger (NO); Boe, Arild, N-4033 Forus (NO); Schmidt, Frank, N-4000 Stavanger (NO); Jacobsen, Endre, N-4050 Sola (NO); Time, Rune W., N-4340 Bryne (NO); Vatne, Harald, N-4350 Narbo (NO)
(74) Representative: Wood, Anthony Charles

(56) References cited:
- DE-A- 2 413 818
- DE-A- 3 447 562
- US-A- 3 635 082
- US-A- 4 074 184
- PATENT ABSTRACTS OF JAPAN, vol. 9, no. 2 (P-325)[1725], 8th January 1985; & JP-A-59 153 161 (HITACHI SEISAKUSHO K.K.) 01-09-1984

## Description

The present invention relates to measurement apparatus and methods in general and, more particularly, to measurement apparatus and methods for measuring flow characteristics of a flowing petroleum stream which may be multi-phase and may include one or more of gas, oil and water.

An object of the present invention is to provide improvements in such method and apparatus.

US-A-3635082 describes apparatus for measuring a flow characteristic of a mixed phase stream, comprising:
a cell of electrically non-conductive material for passage of a mixed phase stream;
first and second sensing means mounted on the cell and spaced a predetermined distance apart along the stream, each said sensing means comprising first and second electrodes forming the plates of a capacitor and adapted to provide a signal representative of the capacitance of the stream as it flows between the electrodes, the first and second electrodes of the first sensing means being substantially the same in size and configuration as the first and second electrodes of the second sensing means;
signal processing means connected to each said sensing means to derive a measure of the velocity of the stream from said signals.

That document particularly describes the use of two substantially identical capacitive sensors spaced a predetermined distance apart along a fluid stream containing finely divided particles, for example coal or iron ore in a stream of compressed air or oxygen.

The present invention is characterized in that:
said apparatus is adapted for measuring a flow characteristic of a flowing petroleum stream, and includes:
third and fourth sensing means mounted on the cell;
said first, second, third and fourth sensing means being spaced apart from one another along the stream;
said third and fourth sensing means each comprising first and second electrodes forming the plates of a capacitor and adapted to provide a signal representative of the capacitance of the stream as it flows between the electrodes, the electrodes of the third and fourth sensing means being disposed in different spatial configurations from one another and from the configuration of the electrodes of the first and second sensing means; and
said signal processing means being connected to all said sensing means to derive a measure of the velocity of the stream from said first and second signals and to derive an indication of the presence and/or relative spatial distribution of at least first and second different fluids within said flowing petroleum stream from said first, third and fourth signals.

The present invention is also characterized by measurement method as set out in claim 5.

An embodiment of the invention will now be described, by way of example, with reference to the accompanying drawings, in which:-
Figure 1 shows a petroleum stream measurement apparatus, constructed in accordance with the present invention, partially in longitudinal section and partially in simplified block diagram form.
Figure 2 shows a more detailed longitudinal section of a portion of a sensing means shown in Figure 1.
Figures 3A, 3B and 3C show different configurations of capacitor plates for the sensing means shown in Figure 1.
Figure 4 is a simplified block diagram of a sensing means shown in Figure 1.

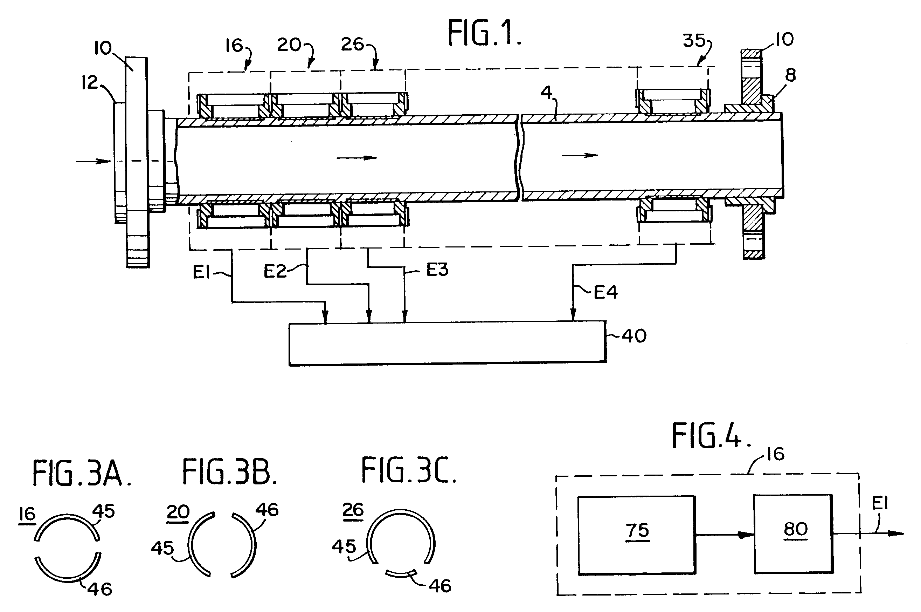

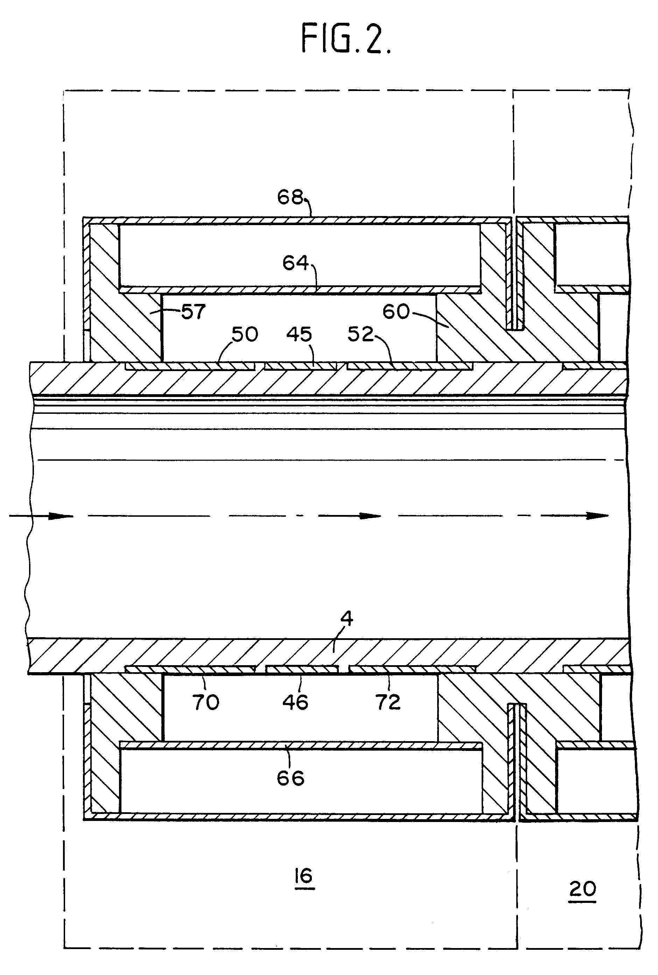

With reference to Figure 1, a measurement cell 4 is made of non-conductive material such as a plastics material, designed so that it can be an integral part of a pipeline carrying a petroleum stream which may be multi-phase and include one or more of gas, oil and water. Monitor cell 4 has fittings 8, 10 and 12 affixed to it for connection with a pipeline (not shown). Capacitive sensing means 16, 20, 26 and 35 are located on the monitor cell 4 and provide signals E1, E2, E3 and E4, respectively, representative of the capacitance of the petroleum stream as it flows through monitor cell 4 as hereinafter explained. Signals E1 to E4 are provided to microcomputing means 40.

With reference to Figure 2, there is shown in detail the sensor portion of sensing means 16. All of the sensing means are the same except for the arrangement of the capacitor plates (electrodes) which will be described more fully hereinafter. Measurement cell 4 has three circumferentially extending grooves 30 for each sensing means. Electrodes 45 and 46 are laid in the center groove and are connected to electronics described in detail hereinafter, but not shown in Figure 2. Further, electrodes 45 and 46 are separated from each other and there is no electrical connection between them. Guards 50, 52, 70, 72 are located in the two other grooves. A protective non-conductive covering of fiberglass tape may be provided to hold the electrodes 45, 46 and guards 50, 52, 70, 72 in place and to protect them from a harsh environment. Electrode 45, and guards 50 and 52 are maintained at a signal ground potential. That is, the amplitudes of signals E1 to E4, are with reference to signal ground. Guards 70 and 72 are held at the same potential as electrode 46.

Also mounted on monitoring cell 4 are mounting blocks 57 and 60. The mounting block 57 is used at the end of a series of sensing means, while mounting block 60 is used internally in the series of sensing means 16 to 26. It should be noted that sensing means 35 utilizes only mounting blocks 57. The purpose of mounting blocks 57 and 60 is to support shielding around the capacitor electrodes 45 and 46. In this regard shield 64 is maintained at the signal ground potential and provides shielding and shield 66 is maintained at the potential of electrode 46. Further, another shield 68 is maintained at an environmental ground such as the pipeline ground to reduce noise on the pipeline from affecting the measurements.

With reference to Figures 3A, 3B and 3C, there are shown the three different arrangements of the capacitor plates respectively of the sensing means 16, 20 and 26.

With regard to Figure 3A and Figure 3B, the capacitor plates are substantially the same except Figure 3B differs by 90° in relationship to Figure 3A. Figure 3C represents the configuration of the capacitor plates of sensing means 26.

In this example sensing means 35 uses the same capacitor plate configuration as sensing means 16 but is spaced a known distance from sensing means 16. This results in signal E4 differing from signal E1 by a time factor related to the velocity of the petroleum stream.

With reference to Figure 4, sensing means 16 includes an RC oscillator means 75. The capacitor for RC oscillator means 75 is formed by the electrodes 45 and 46, mounted on monitor cell 4 with the petroleum stream flowing between electrodes 45 and 46. The resistor (not shown) has a known resistance value and therefore the signal provided by RC oscillator means 75 is related to the capacitance of the dielectric medium between electrodes 45 and 46. Variations in the dielectric constant of the petroleum stream flowing through monitor cell 4 result in corresponding changes in frequency of the oscillator means 75 signal. As the petroleum stream flows between the various capacitor plates, slugs of gas, bubbles of gas, more water, less water and so forth will alter the capacitance between the capacitor plates causing signal E1 to vary accordingly. A phase lock loop means 80 is connected to the oscillator 75 to provide pulse signals whose pulse repetition rate is representative of the sensed capacitance.

It should be noted in an example just given, that sensing means 16 and sensing means 35 have their plates arranged in the same spatial manner but a predetermined distance apart so that the velocity of the stream can now be determined in accordance with the time difference between signals E1 and E4. Microcomputing means 40 is of a conventional type utilizing a microprocessor with associated memories. Stored in the memories are look-up tables for the various values of capacitance associated with different fluid distributions within measurement cell 4. This percent volume of water, gas and oil may be determined using the look-up table memory.

Measurement apparatus and method according to the present invention can provide qualitative information such as capacitance vs. time traces on a graph. The apparatus can measure velocity and length of liquid slugs. The apparatus, for each point of time, can measure approximate cross-sectional fluid distribution by a comparison of signals E1, E2 and E3, and this in turn leads to a determination of volume fractions and hold-ups. (A hold-up in this art is defined as the cross-sectional area of the liquid flow divided by the cross-sectional area of the pipe). Further, the apparatus and method can determine the flow regime of a petroleum stream from the measured cross-sectional distributions and their time evolution. The apparatus can also measure the flow rates of the individual fluids for some flow regimes.

## Claims

1. Apparatus for measuring a flow characteristic of a mixed phase stream, comprising:
a cell (4) of electrically non-conductive material for passage of a mixed phase stream;
first and second sensing means (16,35) mounted on the cell and spaced a predetermined distance apart along the stream, each said sensing means comprising first and second electrodes (45,46) forming the plates of a capacitor and adapted to provide a signal (E1,E4) representative of the capacitance of the stream as it flows between the electrodes, the first and second electrodes of the first sensing means (16) being substantially the same in size and configuration as the first and second electrodes of the second sensing means (35);
signal processing means (40) connected to each said sensing means (16,35) to derive a measure of the velocity of the stream from said signals (E1,E4);
characterised in that:
said apparatus is adapted for measuring a flow characteristic of a flowing petroleum stream, and includes:
third and fourth sensing means (20,26) mounted on the cell;
said first, second, third and fourth sensing means (16,35,20,26) being spaced apart from one another along the stream;
said third and fourth sensing means each comprising first and second electrodes (45,46) forming the plates of a capacitor and adapted to provide a signal (E2,E3) representative of the capacitance of the stream as it flows between the electrodes, the electrodes of the third and fourth sensing means (20,26) being disposed in different spatial configurations from one another and from the configuration of the electrodes of the first and second sensing means (16,35); and
said signal processing means (40) being connected to all said sensing means to derive a measure of the velocity of the stream from said first and second signals (E1,E4) and to derive an indication of the presence and/or relative spatial distribution of at least first and second different fluids within said flowing petroleum stream from said first, third and fourth signals (E1,E2,E3).

2. Apparatus according to Claim 1 characterised in that each said sensing means (16,35,20,26) comprises:
shielding means (64,66) associated with said electrodes (45,46);
an oscillator (75) connected to said electrodes to provide a signal whose frequency at a point in time is representative of the capacitance of the portion of the petroleum stream passing at that time between said first and second electrodes (45,46); and
phase lock loop means (80) connected to said oscillator (75) to provide pulse signals whose pulse repetition rate is representative of said sensed capacitance.

3. Apparatus according to Claim 1 or Claim 2 characterized in that the first and second electrodes (45,46) of the third sensing means (20) are substantially the same in size as the respective first and second electrodes of the first and second sensing means (16,35) but have a configuration displaced by substantially 90° from the configuration of the first and second sensing means about the longitudinal axis of the flowing stream.

4. Apparatus according to any one of Claim 1 to 3 characterised in that the first electrode (45) of the fourth sensing means (26) is larger than its second electrode (46).

5. A method of measuring a flow characteristic of a mixed phase stream, comprising:
causing the stream to flow through a cell of electrically non-conductive material;
sensing the capacitance of the stream as it flows between the first and second electrodes of each of first and second sensing means mounted on the cell and spaced at predetermined distance apart along the stream, the first and second electrodes of the first sensing means being substantially the same in size and configuration as the first and second electrodes of the second sensing means, and providing first and second signals (E1,E4) representative of the respective sensed capacitances; and
processing the first and second signals to derive a measure of the velocity of the stream;
characterised in that:
said method is adapted for measuring a flow characteristic of a flowing petroleum stream, and includes:
sensing the capacitance of the stream as it flows between the first and second electrodes of each of third and fourth sensing means mounted on the cell, said first, second, third and fourth sensing means being spaced apart from one another along the stream, the electrodes of the third and fourth sensing means being disposed in different spatial configurations from one another and from the configuration of the electrodes in the first and second sensing means, and providing third and fourth signals (E2,E3) representative of the respective sensed capacitances; and
said processing step including processing said first and second signals (E1,E4) to derive a measure of the velocity of the stream, and processing said first, third and fourth signals (E1,E2,E3) to derive an indication of the presence and/or relative spatial distribution of at least first and second different fluids within said flowing petroleum stream.

6. A method according to Claim 5 characterised in that each said sensing step comprises:
shielding the electrodes;
providing an oscillatory signal whose frequency at a point in time is representative of the capacitance of the portion of the petroleum stream passing at that time between the first and second electrodes; and
providing pulse signals whose pulse repetition rate is representative of the sensed capacitance.

7. A method according to Claim 5 or Claim 6 characterised in that the sensing step carried out by the third sensing means is carried out utilizing using first and second electrodes of substantially the same size as the respective first and second electrodes of the first and second sensing means but having a configuration displaced by substantially 90° from the configuration of the first and second sensing means about the longitudinal axis of the flowing stream.

8. A method according to any one of Claims 5 to 7 characterised in that the sensing step carried out by the fourth sensing means is carried out utilizing a first electrode larger than the second electrode.

## Patentansprüche

1. Gerät zur Messung einer Strömungseigenschaft eines Mischphasenstroms, umfassend:
eine Zelle (4) aus elektrisch nichtleitendem Material zum Durchgang eines Mischphasenstroms;
ein erstes und zweites Abtastmittel (16, 35), die auf der Zelle angebracht und mit einem vorbestimmten, gegenseitigen Abstand entlang des Stroms angeordnet sind, wobei jedes besagte Abtastmittel eine erste und zweite Elektrode (45, 46) umfaßt, die die Platten eines Kondensators bilden und bestimmt sind, ein Signal (E1, E4) zu liefern, das kennzeichnend für die Kapazität des Stromes ist, wie er zwischen den Elektroden fließt, wobei die erste und zweite Elektrode des ersten Abtastmittels (16) im wesentlichen in Größe und Anordnung gleich der ersten und zweiten Elektrode des zweiten Abtastmittels (35) sind;
Signalverarbeitungsmittel (40), die mit jedem der Abtastmittel (16, 35) verbunden sind, um ein Maß für die Geschwindigkeit des Stroms aus den besagten Signalen (E1, E4) abzuleiten; dadurch gekennzeichnet, daß:
besagtes Gerät bestimmt ist, eine Strömungseigenschaft einer Petroleumströmung zu messen, und umfaßt:
ein drittes und viertes Abtastmittel (20, 26), die auf der Zelle angebracht sind;
wobei besagtes erstes, zweites, drittes und viertes Abtastmittel (16, 35, 20, 26) mit gegenseitigem Abstand entlang des Stroms angeordnet sind;
wobei das dritte und vierte Abtastmittel jeweils eine erste und zweite Elektrode (45, 46) umfaßt, die die Platten eines Kondensators bilden und dazu bestimmt sind, ein Signal (E2, E3) zu liefern, das kennzeichnend für die Kapazität des Stroms ist, wie er zwischen den Elektroden fließt, wobei die Elektroden des dritten und vierten Abtastmittels (20, 26) jeweils in von einander und von der Konfiguration der Elektroden des ersten und zweiten Abtastmittels (16, 35) verschiedenen räumlichen Konfigurationen angeordnet sind; und
besagtes Signalverarbeitungsmittel (40) mit allen besagten Abtastmitteln verbunden ist, um ein Maß für die Geschwindigkeit des Stroms aus besagtem ersten und zweiten Signal (E1, E4) abzuleiten und um einen Hinweis auf das Vorhandensein und/oder die relative räumliche Verteilung von wenigstens einem ersten und zweiten anderen Fluid innerhalb der besagten Petroleumströmung aus besagtem ersten, dritten und vierten Signal (E1, E2, E3) abzuleiten.

2. Gerät nach Anspruch 1, dadurch gekennzeichent, daß jedes besagte Abtastmittel (16, 35, 20, 26) umfaßt:
Abschirmmittel (64, 66), die zu jeder besagten Elektrode (45, 46) gehören;
einen Oszillator (75), der mit besagten Elektroden verbunden ist, um ein Signal zu liefern, dessen Frequenz zu einem Zeitpunkt kennzeichnend für die Kapazität des Anteils des Petroleumstroms ist, der zu der Zeit zwischen besagter erster und zweiter Elektrode (45, 46) durchströmt; und
phasenstarre Schaltkreismittel (80), die mit besagtem Oszillator (75) verbunden sind, um Pulssignale zu liefern, deren Pulswiederholungsrate kennzeichnend ist für besagte abgetastete Kapazität.

3. Gerät nach Anspruch 1 oder 2, dadurch gekennzeichnet, daß die erste und zweite Elektrode (45, 46) des dritten Abtastmittels (20) im wesentlichen von gleicher Größe wie die entsprechende erste und zweite Elektrode des ersten und zweiten Abtastmittels (16, 35) sind, aber eine um im wesentlichen 90° gegenüber der Konfiguration des ersten und zweiten Abtastmittels um die Längsachse der Strömung versetzte Konfiguration aufweisen.

4. Gerät nach einem der Ansprüche 1 bis 3, dadurch gekennzeichnet, daß die erste Elektrode (45) des vierten Abtastmittels (26) größer ist als dessen zweite Elektrode (46).

5. Verfahren zum Messen einer Strömungseigenschaft eines Mischphasenstroms, umfassend:
einen Strom, der durch eine Zelle aus elektrisch nichtleitendem Material fließt;
Abtasten der Kapazität des Stroms, wie er zwischen den ersten und zweiten Elektroden des ersten und zweiten Abtastmittels, die auf der Zelle angebracht und und mit einem vorbestimmten, gegenseitigen Abstand entlang des Stroms angeordnet sind, durchfließt, wobei die erste und zweite Elektrode des ersten Abtastmittels im wesentlichen in Größe und Anordnung gleich der ersten und zweiten Elektrode des zweiten Abtastmittels ist, und Liefern eines ersten und zweiten Signals (E1, E4), die kennzeichnend für die jeweils abgetasteten Kapazitäten sind; und
Verarbeiten des ersten und zweiten Signals, um ein Maß für die Geschwindigkeit des Stroms abzuleiten;
dadurch gekennzeichnet, daß:
besagtes Verfahren dazu bestimmt ist, eine Strömungseigeneigenschaft einer Petroleumströmung zu messen, und einschließt:
Abtasten der Kapazität des Stroms, wie er zwischen den ersten und zweiten Elektroden des dritten und vierten Abtastmittels, die auf der Zelle angebracht sind, durchfließt, wobei besagtes erstes, zweites, drittes und viertes Abtastmittel mit gegenseitigem Abstand voneinander entlang des Stroms angeordnet sind, wobei die Elektroden des dritten und vierten Abtastmittels jeweils in von einander und von der Konfiguration der Elektroden des ersten und zweiten Abtastmittels verschiedenen räumlichen Konfigurationen angeordnet sind, und Liefern eines dritten und vierten Signals (E2, E3), die kennzeichnend für die abgetasteten Kapazitäten sind; und
wobei besagter Verarbeitungsschritt das Verarbeiten des besagten ersten und zweiten Signals (E1, E4) einschließt, um ein Maß für die Geschwindigkeit des Stroms abzuleiten, und das Verarbeiten besagten ersten, dritten und vierten Signals (E1, E2, E3), um einen Hinweis auf das Vorhandensein und/oder die relative räumliche Verteilung von wenigstens einem ersten und zweiten anderen Fluid innerhalb besagter Petroleumströmung abzuleiten.

6. Verfahren nach Anspruch 5, dadurch gekennzeichnet, daß jeder besagte Abtastschritt umfaßt:
Abschirmen der Elektroden;
Liefern eines Oszillatorsignals, dessen Frequenz zu einem Zeitpunkt kennzeichnend ist für die Kapazität des Anteils des Petroleumstroms, der zu der Zeit zwischen der ersten und zweiten Elektrode durchgeht; und
Liefern von Pulssignalen, deren Pulswiederholungsrate kennzeichnend ist für die abgetastete Kapazität.

7. Verfahren nach Anspruch 5 oder 6, dadurch gekennzeichnet, daß der durch das dritte Abtastmittel ausgeführte Abtastschritt unter Verwendung einer ersten und zweiten Elektrode ausgeführt wird, die im wesentlichen die gleiche Größe wie die entsprechenden ersten und zweiten Elektroden des ersten und zweiten Abtastmittels, aber eine um im wesentlichen 90° gegenüber der Konfiguration des ersten und zweiten Abtastmittels um die Längsachse der Strömung versetzte Konfiguration haben.

8. Verfahren nach einem der Ansprüche 5 bis 7, dadurch gekennzeichnet, daß der durch das vierte Abtastmittel ausgeführte Abtastschritt unter Verwendung einer ersten Elektrode, die größer als die zweite Elektrode ist, ausgeführt wird.

## Revendications

1. Appareil pour la mesure d'une caractéristique fluidique d'un courant à phases mélangées, comprenant:
une cellule (4) en matériau non-conducteur de l'électricité, permettant le passage d'un courant à phases mélangées;
des premier et deuxième moyens de mesure (16, 35) montés sur la cellule, et espacés d'une distance prédéterminée entre eux le long du courant, chaque dit moyen de mesure comprenant des première et seconde électrodes (45, 46), formant les armatures d'un condensateur et adaptées pour produire un signal (E1, E4) représentant la capacité du courant lorsqu'il s'écoule entre les électrodes, les première et seconde électrodes du premier moyen de mesure (16) présentant sensiblement la même taille et configuration que les première et seconde électrodes du deuxième moyen de mesure (35);
un moyen de traitement de signaux (40) relié à chaque dit moyen de mesure (16, 35), pour dériver une mesure de la vitesse du courant à partir desdits signaux (E1, E4);
caractérisé en ce que:
ledit appareil est adapté pour mesurer une caractéristique fluidique d'un courant de pétrole qui s'écoule, et comprend:
des troisième et quatrième moyens de mesure (20, 26) montés sur la cellule;
lesdits premier, deuxième, troisième et quatrième moyens de mesure (16, 35, 20, 26) étant espacés entre eux le long du courant;
lesdits troisième et quatrième moyens de mesure comprenant chacun des première et seconde électrodes (45, 46), formant les armatures d'un condensateur et adaptées pour produire un signal (E2, E3) représentant la capacité du courant lorsqu'il s'écoule entre les électrodes, les électrodes des troisième et quatrième moyens de mesure (20, 26) étant disposées selon des configurations spatiales différentes les unes par rapport aux autres, et par rapport à la configuration des électrodes des premier et deuxième moyens de mesure (16, 35); et
ledit moyen de traitement de signaux (40) étant connecté à tous lesdits moyens de mesure, pour dériver une mesure de la vitesse du courant à partir desdits premier et second signaux (E1, E4), et pour dériver une indication de la présence et/ou la distribution spatiale relative d'au moins des premier et deuxième fluides différents, contenus dans ledit courant de pétrole qui s'écoule, à partir desdits premier, troisième et quatrième signaux (E1, E2, E3).

2. Appareil selon la revendication 1, caractérisé en ce que chaque dit moyen de mesure (16, 35, 20, 26) comprend:
des moyens d'écrantage (64, 66) associés auxdites électrodes (45, 46);
un oscillateur (75) relié auxdites électrodes, pour produire un signal dont la fréquence à un point donné dans le temps représente la capacité de la partie du courant de pétrole passant à ce moment entre lesdites première et seconde électrodes (45, 46); et
un moyen servant de boucle à phase asservie (80) relié audit oscillateur (75), pour produire des signaux impulsionnels dont la vitesse de répétition d'impulsion représente ladite capacité mesurée.

3. Appareil selon la revendication 1 ou 2, caractérisé en ce que les première et seconde électrodes (45, 46) du troisième moyen de mesure (20) présentent sensiblement la même taille que les première et seconde électrodes respectives des premier et deuxième moyens de mesure (16, 35), mais présentent une configuration déplacée de pratiquement 90° par rapport à la configuration des premier et deuxième moyens de mesure, autour de l'axe longitudinal du courant en écoulement.

4. Appareil selon l'une quelconque des revendications 1 à 3, caractérisé en ce que la première électrode (45) du quatrième moyen de mesure (26) est plus grande que sa seconde électrode (46).

5. Procédé de mesure d'une caractéristique fluidique d'un courant à phases mélangées, comprenant:
la provocation de l'écoulement du courant à travers une cellule en matériau non-conducteur de l'électricité;
la mesure de la capacité du courant lorsqu'il s'écoule entre les première et seconde électrodes de chacun desdits premier et deuxième moyens de mesure, montés sur la cellule et espacés d'une distance prédéterminée entre eux le long du courant, les première et seconde électrodes du premier moyen de mesure présentant sensiblement la même taille et configuration que les première et seconde électrodes du deuxième moyen de mesure, et produisant des premier et deuxième signaux (E1, E4) représentant les capacités mesurées respectives; et
le traitement des premier et deuxième signaux, pour dériver une mesure de la vitesse de l'écoulement;
caractérisé en ce que:
ledit procédé est adapté pour mesurer une caractéristique fluidique d'un courant de pétrole en écoulement, et comprend:
la mesure de la capacité du courant lorsqu'il s'écoule entre les première et seconde électrodes de chacun desdits troisième et quatrième moyens de mesure, montés sur la cellule, lesdits premier, deuxième, troisième et quatrième moyens de mesure étant espacés entre eux le long du courant, les électrodes des troisième et quatrième moyens de mesure étant disposées selon des configurations spatiales différentes les unes par rapport aux autres, et par rapport à la configuration des électrodes des premier et deuxième moyens de mesure, et produisant des troisième et quatrième signaux (E2, E3) représentant les capacités mesurées respectives; et
ladite étape de traitement comprenant le traitement desdits premier et deuxième signaux (E1, E4), pour dériver une mesure de la vitesse du courant, et le traitement desdits premier, troisième et quatrième signaux (E1, E2, E3) pour dériver une indication de la présence et/ou la distribution spatiale relative d'au moins des premier et deuxième fluides différentes contenus dans ledit courant de pétrole s'écoulant.

6. Procédé selon la revendication 5, caractérisé en ce que chaque étape de mesure comprend:
l'écrantage des électrodes;
la production d'un signal oscillant dont la fréquence à un point donné dans le temps indique la capacité de la partie du courant de pétrole passant à ce moment entre les première et seconde électrodes; et
la production de signaux impulsionnels dont la vitesse de répétition d'impulsion représente la capacité mesurée.

7. Procédé selon la revendication 5 ou 6, caractérisé en ce que l'étape de mesure réalisée par le troisième moyen de mesure est effectuée en utilisant des première et seconde électrodes dotées sensiblement de la même taille que les première et seconde électrodes respectives des premier et deuxième moyens de mesure, mais présentant une configuration déplacée de pratiquement 90° par rapport à la configuration des premier et deuxième moyens de mesure, autour de l'axe longitudinal du courant en écoulement.

8. Procédé selon l'une quelconque des revendications 5 à 7, caractérisé en ce que l'étape de mesure réalisée par le quatrième moyen de mesure est effectuée en utilisant une première électrode plus grande que la seconde électrode.
